# EUROPEAN PATENT SPECIFICATION

(11) **EP 2 703 037 B1**
(45) Date of publication and mention of the grant of the patent: **27.04.2016**
(21) Application number: 12799938.1
(22) Date of filing: 08.05.2012
(51) Int. Cl.: A61M 25/09, A61B 1/00

(54) **INTRACORPOREAL INSERTION INSTRUMENT**
INTRAKOPORALES EINFÜHRINSTRUMENT
INTRACORPORELLES INTRUMENT D'INSERTION

(30) Priority: 16.06.2011 JP 2011134500
(43) Date of publication of application: 05.03.2014
(73) Proprietor: Olympus Corporation, Shibuya-ku Tokyo (JP)
(72) Inventor: HINO Kazuhiko, Tokyo 151-0072 (JP)
(74) Representative: Schicker, Silvia
(86) International application number: PCT/JP2012/061767
(87) International publication number: WO 2012/172885

(56) References cited:
- EP-A2- 0 773 037
- WO-A1-2006/117937
- JP-A- 2003 093 516
- JP-A- 2010 000 351
- JP-A- 2011 500 191
- JP-U- H0 636 603
- US-A1- 2006 271 090
- US-A1- 2009 036 834

## Description

### Technical Field

The present invention relates to an intracorporeal insertion instrument.

### Background Art

Conventionally, intracorporeal insertion instruments have been used for various examinations and various treatments in medical fields. Intracorporeal insertion instruments include a catheter for CTO (chronic total occlusion), a guide wire for allowing passage through a narrowed area in a bile duct or a pancreatic duct, or the like.

For example, in ERCP (endoscopic retrograde cholangiopancreatography), an endoscope is used, and a contrast agent is injected from the duodenal papilla, to confirm the narrowed area of the bile duct while viewing an X-ray image, and treatment for expanding the narrowed area of the bile duct by inserting a stent and the like into the narrowed area using a guide wire.

In this case, the intracorporeal insertion instrument is inserted close to such a narrowed area. Since the bile duct or the like intricately branches off, it is not easy for a surgeon to select one branched duct having a narrowed area among a plurality of branched ducts which branch off in a plurality of directions and insert and place a guide wire in the selected branched duct, while viewing an X-ray image. Same is true for a catheter used for a therapy of CTO (chronic total occlusion).

Japanese Unexamined Patent Application Publication (Translation of PCT Application) No. 2008-509718 discloses a technique for previously forming a guide wire, which passes through a guide tube in a catheter to protrude from a distal end, such that the guide wire bends when the guide wire is projected from a narrow guide tube and released, in order to advance the guide wire in the lumen in a direction of a desired duct at a branch point.

Further, Japanese Unexamined Patent Application Publication (Translation of PCT Application) No. 2011-500191 proposes a catheter tool which is capable of changing the moving direction of the guide wire in order to orient the guide wire projected from a distal end portion in a desired branching direction. In the proposal, the catheter tool has a mechanism for changing the separating distance between two guide wires, in order to change the moving direction of the guide wires. For example, a balloon mechanism and the like for controlling the separating distance between the two guide wires is provided at a distal end portion of the catheter tool.

However, as in the catheter tool according to the first proposal, even if the bending habit is previously applied to the guide wire, a plurality of guide wires are not necessarily bent in different directions when the guide wires are released from a distal end portion of the catheter. This is because the guide wire rotates in the axial direction of the guide wire in the guide tube of the catheter and a direction of the bending habit of the guide wire is indefinite when the guide wire is protruded from the distal end portion of the catheter.

Further, in the case of the catheter tool according to the second proposal, a configuration for changing the separating distance between the two guide wires is required for changing the moving direction of the guide wires, and therefore there arises a problem that the configuration is complicated to result in making a diameter of the catheter tool thick. Furthermore, since the surgeon is required to perform an operation for inflation of the balloon, the surgeon has to perform, while holding the catheter tool by one hand, the inflation operation of the balloon and the insertion operation of the guide wires by the other hand, so that operability is not good.

Therefore, the present invention has been made in view of the foregoing problems and an object of the present invention is to provide an intracorporeal insertion instrument which is simple in configuration and suitable for diameter reduction, and is configured such that a plurality of guide wires are protruded in directions different from each other.

EP 0 773 037 A2 discloses a stylet unit which comprises a flexible tubular stylet sleeve and an inner stylet arranged to move freely inside a channel formed in the sleeve. The stylet sleeve has a proximal end and a distal end, and the stylet has a proximal end and a distal end. In an area before its distal end, the inner stylet has a pre-bent distal end section which is shaped like a semi-circle. Interacting parts or segments of the stylet and the channel formed in the sleeve may have non-circular cross-sections and may be devised as concentric ovals, ellipses or regular polygons with rounded corners.

US 2009/0036834 A1 discloses a guide wire which comprises a sleeve and a core. A cross-section of the core varies along a longitudinal axis of the core.

US 2006/0271090 A1 relates to a catheter system which includes a main elongated element and an auxiliary elongated element. The main elongated element may be a catheter having a proximal end and a distal end. Spring wires which are used as a stopper element are designed to lay substantially horizontal to the catheter in an unextended position and coil or spring into a stopper upon release.

### Disclosure of Invention

### Means for Solving the Problem

The problem is solved by an intracorporeal insertion instrument as defined in independent claim 1.

### Brief Description of the Drawings

FIG. 1 relates to a first embodiment of the present invention, and illustrates a state where a guide wire is inserted in a desired direction at one branch point in a bile duct in ERCP (Endoscopic retrograde cholangiopancreatography).
FIG. 2 is a front view illustrating a configuration of an intracorporeal insertion instrument 21 according to the first embodiment of the present invention.
FIG. 3 is a cross-sectional view of the intracorporeal insertion instrument 21 in FIG. 2, which is taken along the III-III line.
FIG. 4 is a perspective view illustrating a state of the guide wires 25, 26 protruded from a distal end portion 22a of a sheath main body 22 according to the first embodiment of the present invention.
FIG. 5 is a perspective view illustrating proximal-end openings 23tb, 24tb, diameters of which are expanded at a proximal end portion 22b of the sheath main body 22, according to the first embodiment of the present invention.
FIG. 6 is a front view of a configuration of an intracorporeal insertion instrument according to a modified example 1 of the first embodiment of the present invention.
FIG. 7 illustrates the proximal-end opening viewed from the proximal end side shown by the arrow Vp in FIG. 6.
FIG. 8 illustrates an intracorporeal insertion instrument according to a modified example 2 of the first embodiment of the present invention.
FIG. 9 illustrates another example of the shapes of the distal-end openings of the sheath main body and the shapes of the guide wires, according to a modified example 3 of the first embodiment of the present invention.
FIG. 10 illustrates yet another example of the shapes of the distal-end openings of the sheath main body and the shapes of the guide wires, according to the modified example 3 of the first embodiment of the present invention.
FIG. 11 is a cross-sectional view of an intracorporeal insertion instrument 21 according to a second embodiment of the present invention.

### Best Mode for Carrying Out the Invention

Hereinafter, embodiments of the present invention will be described with reference to drawings.

Note that, in the drawings used in the description below, a different scale size is used for each of the components in order to allow each of the components to be illustrated in a recognizable size in the drawings, and the present invention is not limited to the number, shapes, ratio of the sizes of the components, and a relative positional relationship among the components shown in these drawings.

### (First Embodiment)

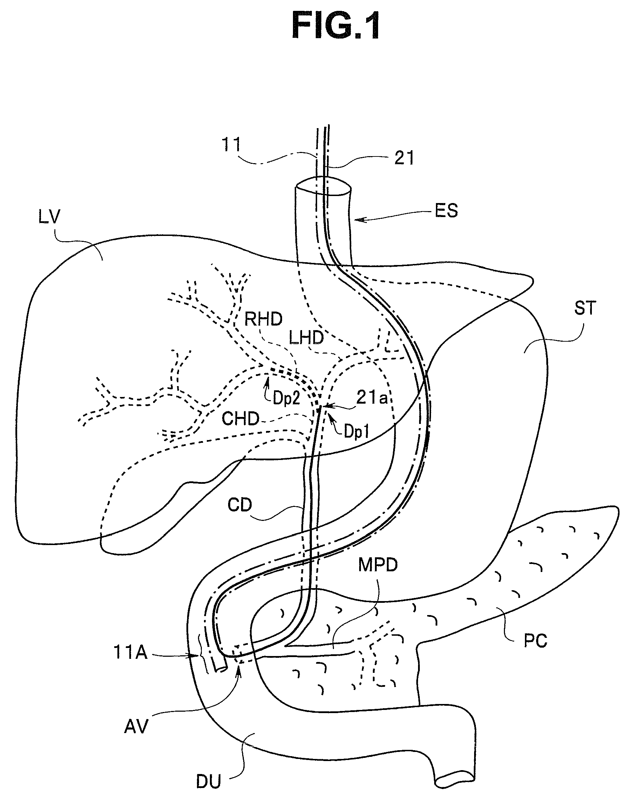

The present embodiment will be described using an example of an intracorporeal insertion instrument including a plurality of guide wires for a bile duct and a sheath main body through which the plurality of guide wires are inserted. FIG. 1 illustrates a state where a guide wire is inserted in a desired direction at one branch point in a bile duct in ERCP (endoscopic retrograde cholangiopancreatography).

FIG. 1 illustrates a state where a distal end portion 11A of an insertion portion 11 of an endoscope inserted from the mouth of a subject passes through the esophagus ES and the stomach ST to reach in the vicinity of the papilla Vater AV of the duodenum DU. In a treatment instrument insertion channel inserted in the insertion portion 11 of the endoscope, an intracorporeal insertion instrument 21 for bile duct is inserted. The endoscope is a lateral-view endoscope, and is configured such that the intracorporeal insertion instrument 21 can be protruded from an opening portion of the distal end portion 11A of the insertion portion 11.

A surgeon operates a raising stand of the distal end portion 11A while viewing an image displayed on a monitor (not shown) of the endoscope apparatus, thereby capable of guiding the distal end of the intracorporeal insertion section 21 to an orifice of the papilla Vater AV. In FIG. 1, the intracorporeal insertion instrument 21 passes from the papilla Vater AV through the common bile duct CD not through the main pancreatic duct MPD. The surgeon passes the intracorporeal insertion instrument 21 through the common bile duct CD, and further enables a distal end portion 21 a of the intracorporeal insertion instrument 21 to advance toward the liver LV while viewing the X-ray image. A distal end portion of the common hepatic duct CHD on the distal end side of the common bile duct CD branches off into the right hepatic duct RHD and the left hepatic duct LHD, and the respective tips of the right hepatic duct RHD and the left hepatic duct LHD further branch off. The bile duct finely branches off and forms a dendritic network, to be a bile capillary in the liver LV.

FIG. 1 illustrates a state where the distal end portion 21a of the intracorporeal insertion instrument 21 is located at a branch point Dp1 between the right hepatic duct RHD and the left hepatic duct LHD in the common hepatic duct CHD.

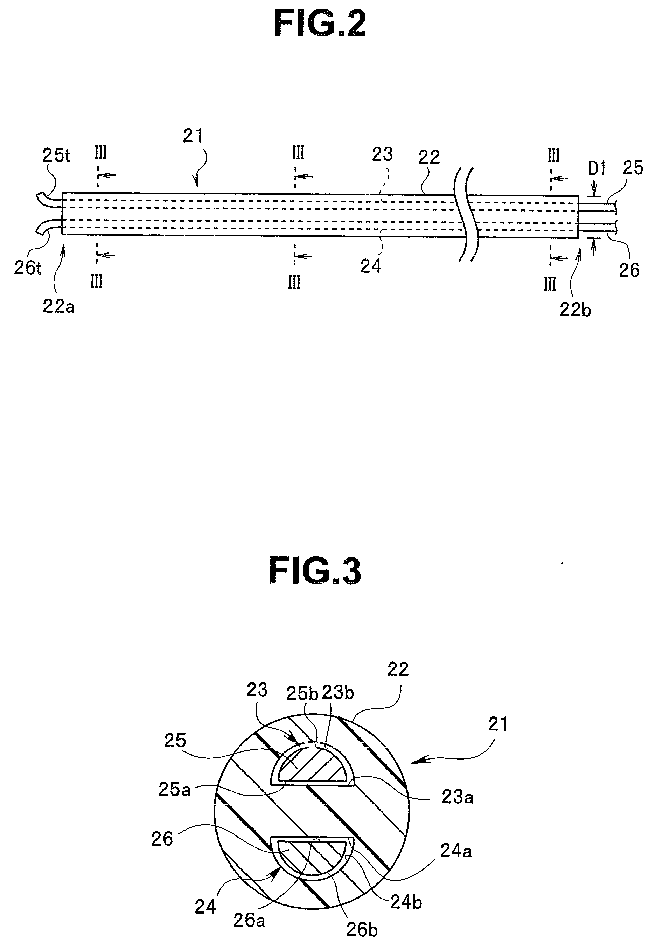

FIG. 2 is a front view illustrating a configuration of the intracorporeal insertion instrument 21. The intracorporeal insertion instrument 21 is configured by including a sheath main body 22 which is an elongated, that is, long double lumen tube, and two guide wires 25, 26 inserted into two lumens 23, 24 which penetrate through from a proximal end portion 22b to a distal end portion 22a of the sheath main body 22. That is, the lumens 23, 24 are formed to allow the respective guide wires as guide tools to be inserted therethrough, and to be able to be protruded from the distal-end openings of the sheath main body 22.

Note that the sheath main body 22 is a double lumen tube having two lumens in the present embodiment, but may be a multiple lumen tube having three or more lumens.

The sheath main body 22 has a circular cross section and includes inside thereof the two lumens 23, 24 formed along the axis of the sheath main body 22. A diameter D1 of the circle of the cross section perpendicular to the axis direction of the sheath main body 22 is 1.2 mm, for example, and the material of the sheath main body is a fluorine resin such as PTFE (polytetrafluoroethylene).

FIG. 3 is a cross-sectional view of the intracorporeal insertion instrument 21 in FIG. 2, which is taken along the III-III line. As shown in FIG. 3, the intracorporeal insertion instrument 21 is configured such that cross sections thereof have the same cross-sectional shape at any position. The cross sections of the respective lumens 23 and 24, which are taken along the direction perpendicular to the axis of the sheath main body 22, have a non-circular shape, i.e., a semicircular shape in the present embodiment.

As shown in FIG. 3, the two lumens 23 and 24 are formed such that inner walls of two plane portions 23a, 24a corresponding to the straight parts of the semicircular shapes are parallel with each other when the sheath main body 22 is straightened. The sheath main body 22 has a length about two meters, for example. The sheath main body 22 including inside thereof such two lumens 23, 24 can be produced by the publicly-known extrusion molding method.

The two guide wires 25, 26 are elongated and elastic wires made of stainless steel and have semicircular cross-sectional shapes which are similar to the cross-sectional shapes of the lumens 23, 24 and have a size insertable into the lumens 23, 24. That is, the guide wires 25, 26 have shapes complementary to the cross-sectional shapes of the lumens 23, 24. Therefore, as shown in FIG. 3, the guide wires 25, 26 respectively have flat portions 25a, 26a and cylindrical surfaces 25b, 26b. The surfaces of the guide wires 25, 26 are coated by fluorine coating, for example.

Furthermore, the guide wires 25, 26 have cross-sectional shapes having a size capable of preventing the respective guide wires from rotating around the axes of the guide wires in the respective lumens. Therefore, only slight gaps exist between the guide wires 25, 26 and the inner walls of the lumens 23, 24. The lengths of the guide wires 25, 26 are equal to or longer than two meters, for example.

Such guide wires 25, 26 can be formed using a die by passing circular cylinder-shaped members through a semicircular hole of the die and pulling the members. Alternatively, the guide wires 25, 26 as shown in FIG. 3 can be produced also by pulling and extending semicircular cylinder-shaped members.

Note that the guide wires 25, 26 as guide tools may be tubular wires, instead of solid wires.

A bending habit is previously applied to the distal end portions 25t, 26t of the guide wires 25, 26. In the present embodiment, the bending habit is applied to the respective distal end portions 25a, 26a of the guide wires 25, 26 along the axis direction of the respective guide wires such that the distal end portions 25a, 26a bend toward the cylindrical surfaces 25b, 26b of the guide wires 25, 26, respectively.

The bending habit can be applied by manual operation by a human, for example, by fixing the distal end portions 25t, 26t of the guide wires 25, 26 on a fixing table and drawing the cylindrical surfaces 25b, 26b of the distal end portion 25t, 26t along the axes of the guide wires 25, 26 while pressing a predetermined jig on the cylindrical surfaces.

Note that the bending habit may be applied over the entire of the guide wires 25, 26, or may be applied at least to the distal end portions 25t, 26t of the guide wires 25, 26. Therefore, the guide wires 25, 26 as guide tools are elongated members configured such that the cross-sectional shapes of at least the distal ends are non-circular and the bending habit along the axis direction is applied to at least the distal end portions.

The guide wires 25, 26 are inserted from the respective proximal-end openings of the lumens 23, 24, which are located at the proximal end portion 22b of the sheath main body 22. The shapes of the two proximal-end openings are the same as the cross-sectional shapes of the lumens 23, 24. The guide wires 25, 26 are thin, made of stainless steel, and have elasticity. Therefore, even if the distal end portions 25t, 26t are bent, the guide wires can be easily inserted into the lumens 23, 24 from the respective proximal-end openings.

When the guide wires 25, 26 are inserted into the lumens 23, 24, respectively, and the distal end portions 25t, 26t are protruded from the distal end portion 22a of the sheath main body 22, the distal end portions 25t, 26t of the guide wires 25, 26 do not contact the inner walls of the lumens 23, 24 to be released, thereby allowing the distal end portions 25t, 26t of the guide wires 25, 26 to bend respectively in the directions in which the bending habit is applied.

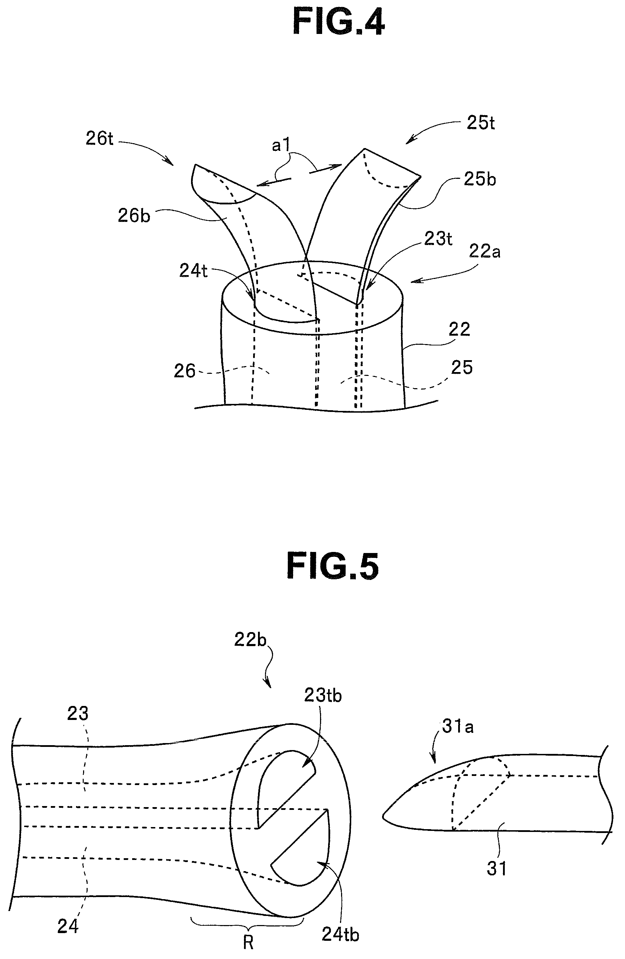

FIG. 4 is a perspective view illustrating the state of the guide wires 25, 26 protruded from the distal end portion 22a of the sheath main body 22. As shown in FIG. 4, if the guide wires 25, 26 protrude from the distal-end openings 23t, 24t of the lumens 23, 24, the distal end portion 25t, 26t of the guide wires 25, 26 bend toward the cylindrical surfaces 25b, 26b which are the directions in which the bending habit is previously applied. Therefore, the distal end portions 25a, 26a protrude in directions different from each other.

The distal-end openings 23t, 24t of the lumens 23, 24 at the distal end portion 22a are formed such that the flat surface 25a of the guide wire 25 and the flat surface 26a of the guide wire 26 are opposed to each other at the distal end portion 22a of the sheath main body 22. Therefore, when the guide wires 25, 26 protrude from the distal end portion 22a of the sheath main body 22, the guide wires surely bend in the previously-determined directions different from each other.

The distal-end openings 23t, 24t are formed so as to prevent the distal end portions 25t, 26t of the guide wires 25, 26 from rotating around the axes thereof. Further, the distal-end openings 23t, 24t are formed such that the distal end portions 25t, 26t are separated from each other as shown by the arrow a1 along the protruding directions.

That is, the sheath main body 22 includes a plurality of lumens 23, 24 configured such that a plurality of guide wires 25, 26 are inserted from the plurality of proximal-end openings provided at the proximal end portion 22b and the plurality of guide wires 25, 26 can be protruded from the plurality of distal-end openings 23t, 24t provided at the distal end portion 22a. Furthermore, the plurality of distal end openings 23t, 24t are formed at the distal end portion 22a such that the respective guide wires do not rotate around the axes thereof in the distal-end openings and the plurality of distal end portions 25t, 26t of the plurality of guide wires 25, 26 are separated from each other along the protruding directions.

Therefore, the directions in which the two guide wires 25, 26 bend are determined by the positions and orientations of the distal-end openings 23t, 24t of the two lumens 23, 24 at the distal end portion 22a of the sheath main body 22, and the directions of bending caused by the bending habit previously applied to the guide wires 25,26.

In the above-described example, the bending habit is previously applied to the respective guide wires 25, 26 so as to bend toward the cylindrical surfaces 25b, 26b, and at the distal end portion 22a of the sheath main body 22, the distal-end openings 23t, 24t of the lumens 23, 24 are formed such that the flat surfaces 25a, 26a of the respective guide wires 25, 26 are opposed to and parallel with each other. Therefore, as shown in FIG. 4, the distal end portions 25t, 26t of the two guide wires 25, 26, which protrude from the distal end portion 22a, bend in the directions separated from each other as shown by the arrow a1 as going toward the distal end direction in the axis direction of the sheath main body 22.

For example, as shown in FIG. 1, when the distal end portion 21a of the intracorporeal insertion instrument 21 is at the branch point between the right hepatic duct RHD and the left hepatic duct LHD, the distal end portions 25t, 26t of the guide wires 25, 26 protrude in different directions, which allows the surgeon to easily pass either of the guide wires in a desired bile duct.

Furthermore, as shown by the dotted lines in FIG. 1, for example, the distal end portions 25t, 26t of the guide wires 25, 26 protrude in different directions also at a branch point Dp2, which is located further ahead, in the right hepatic duct RHD. Therefore, it is easy for the surgeon to introduce the intracorporeal insertion instrument 21 to a desired site.

Note that the cross-sectional shapes of the lumens 23, 24 of the sheath main body 22 are the same from the proximal end portion 22b to 22a. However, as shown in FIG. 4, in order to allow the two guide wires 25, 26 to surely bend in different directions at the distal end portion 22a, it is sufficient that the guide wires do not rotate around the axes thereof at the distal-end openings 23t, 24t of the sheath main body 22. Therefore, the cross-sectional shapes of the lumens 23, 24 are not necessarily the same from the proximal end portion 22b to the distal end portion 22a.

As described above, according to the present embodiment, it is possible to provide an intracorporeal insertion instrument which has a simple configuration and is suitable for diameter reduction, and which enables a plurality of guide wires to protrude in the directions different from each other. As a result, the surgeon can rapidly insert the guide wire to a desired position by using such an intracorporeal insertion instrument 21, which reduces a burden on the surgeon and the patient.

In addition, since the above-described intracorporeal insertion instrument has a simple structure, the instrument can be produced at reduced cost. Furthermore, since the above-described intracorporeal insertion instrument has a simple structure, cleaning is easy and such an intraporporeal insertion instrument is suitable for reuse.

Note that the opening area of the proximal-end openings 23tb and 24tb may be formed to be large at the proximal end portion 22b of the sheath main body 22 in order to facilitate the insertion of the guide wires 25, 26 into the proximal-end openings 23tb, 24tb, respectively. In other words, each of the plurality of lumens 23, 24 has cross-sectional shapes which are different between the distal end portion 22a and the proximal end portion 22b of the sheath main body 22, and the proximal-end openings of the respective lumens may be made larger than the distal-end openings of the respective lumens.

FIG. 5 is a perspective view for illustrating the proximal-end openings 23tb, 24tb, diameters of which are expanded at a proximal end portion 22b of the sheath main body 22. As shown in FIG. 5, the respective cross-sectional areas of the proximal-end openings 23tb, 24tb are larger than the respective cross-sectional areas of the lumens 23, 24 in the sheath main body 22. That is, the lumens 23, 24 are formed such that the cross-sectional areas of the respective lumens gradually become larger toward the proximal-end openings 23tb, 24tb in a predetermined range R of the proximal end portion 22b.

As shown in FIG. 5, in such lumens 23, 24, the proximal-end openings 23tb, 24tb, the opening areas of which are made large, can be formed by inserting a jig 31 having a tapered distal end portion into the respective lumens 23, 24 from the proximal end portion 22b of the sheath main body 22. A tapered portion 31a of the jig 31 has a part larger than the cross-sectional areas of the lumens 23, 24 in the sheath main body 22.

In other words, the cross-sectional shape of each of the lumens in the sheath main body 22 has a part which gradually changes from the proximal-end opening to the distal-end opening, the shape gently changes from the proximal end portion 22b to the distal end portion 22a, and the diameter of each of the lumens becomes smaller from the proximal end portion 22b to the distal end portion 22a.

According to the proximal-end openings 23tb, 24tb, the opening areas of which are formed to be large, it is easy for the user to insert the guide wires 25, 26 into the lumens 23, 24.

Hereinafter, modified examples of the present embodiment are described.

### (Modified Example 1)

As shown in FIG. 2, the sheath main body in the above-described embodiment is one sheath formed from the distal end portion 22a to the proximal end portion 22b. However, the intracorporeal insertion instrument in the modified example 1 includes on the proximal end portion side thereof a plurality of sheath portions branched off for each of the lumens.

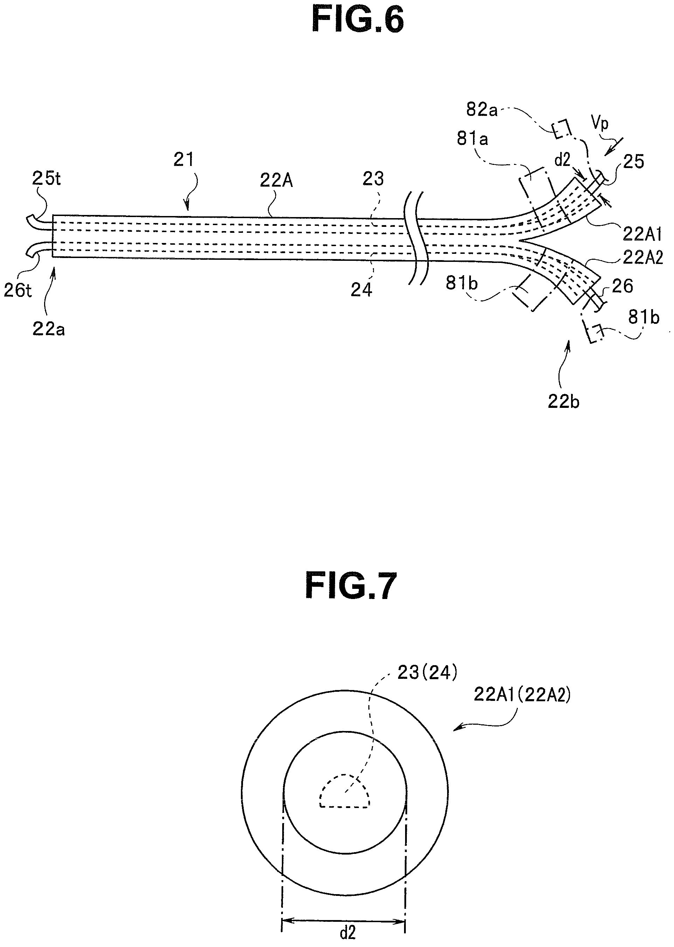

FIG. 6 is a front view of a configuration of the intracorporeal insertion instrument according to the present modified example. The proximal end portion 22b of a sheath main body 22A according to the present modified example is bifurcated, as shown in FIG. 6. That is, the proximal end portion 22b includes sheath portions 22A1, 22A2 which are branched off for each of the lumens. The sheath portion 22A1 is the sheath portion of the lumen 23, and the sheath portion 22A2 is the sheath portion of the lumen 24. The lengths of the sheath portions 22A1, 22A2 at the bifurcated part is about 20 to 30 cm, for example.

The proximal end portion 22b of the sheath main body 22A is provided with the sheath portions corresponding to the respective lumens. Therefore, it is easy for the user such as a surgeon to insert the guide wires 25, 26 into the respective lumens 23, 24.

Furthermore, also in the present modified example, the lumens 23, 24 at the proximal end portion 22b may be expanded so as to facilitate the insertion of the guide wires 25, 26.

FIG. 7 illustrates the proximal-end opening viewed from the proximal end side shown by the arrow Vp in FIG. 6. As shown in FIG. 7, since the inner diameter d2 of the proximal-end openings of the sheath portions 22A1, 22A2 is expanded, it is easy for the user to insert the guide wires 25, 26 into the lumens 23, 24, respectively. The inner diameters of the proximal-end openings can be expanded by using the above-described jig 31 having a tapered portion whose cross section is a circular shape.

### (Modified Example 2)

An intracorporeal insertion instrument according to the modified example 2 is formed such that the user can distinguish between the flat portion and the cylindrical surface when inserting the guide wires from the proximal end portion 22b. The user can discriminate the respective orientations of the guide wires and the sheath main body. Therefore, the user can easily insert the guide wires into the lumens.

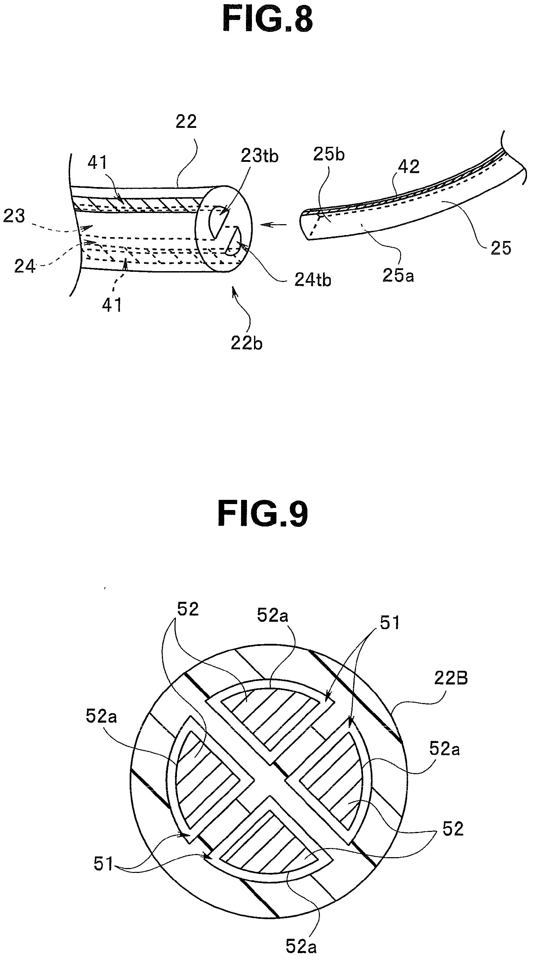

FIG. 8 illustrates the intracorporeal insertion instrument according to the present modified example. On the outer circumferential side of the sheath main body 22 of the proximal end portion 22b, a linear colored portion 41 whose color is different from the color of the outer circumferential surface of the sheath main body 22 is provided on the side of each of the cylindrical surfaces 23b, 24b of the lumens along the axis direction of the sheath main body 22. Similarly, a linear colored portion whose color is different from the color of the guide wire 25 is provided also on the cylindrical surface 25b of the guide wire 25.

Specifically, the two linear colored portions 41 are provided on at least the outer circumferential surface of the proximal end portion 22b of the sheath main body 22. As shown in FIG. 3, the respective lumens 23, 24 are formed such that the cylindrical surfaces 23b, 24b face toward the outer circumferential side of the sheath main body 22. Accordingly, the colored portions 41 are provided at the positions close to the top portions of the cylindrical surfaces of the respective lumens.

Also, the colored portions 42 are provided to the guide wires 25, 26, along the top portions of the cylindrical surfaces 25b, 26b.

Note that the colored portions 41, 42 can be provided to the sheath main body 22 or the guide wires 25, 26 by applying paint of a predetermined color.

That is, the colored portions 41 are provided on the outer circumferential surface of the sheath main body 22 along the vicinity of the top portions of the parts forming circular arcs on the cross sections of the respective lumens 23, 24, and also the colored portions 42 are provided to the guide wires 25, 26, along the top portions of the parts forming circular arcs on the cross sections of the guide wires. In other words, the colored portions 41, 42 are provided to the sheath main body 22 and the respective guide wires, as the indicators for positioning in the circumferential direction of the insertion axis of the respective guide wires at the proximal-end openings of the sheath main body 22.

As a result, if the user inserts the guide wires 25, 26 from the proximal end portion 22b of the sheath main body 22 such that the colored portions 41 and 42 overlap each other on a straight line, when viewing the sheath main body 22 and the guide wires 25, 26, the user can insert the guide wires 25, 26 having non-circular cross-sectional shapes into the proximal-end openings 23tb, 24tb having non-circular cross-sectional shapes. Therefore, the user can easily insert the guide wires into the lumens of the sheath main body.

### (Modified Example 3)

In the above-described embodiment and the respective modified examples, the shape of each of the distal-end openings and the cross-sectional shape of each of the distal end portions of the guide wires are semicircular shape, but may be other shapes.

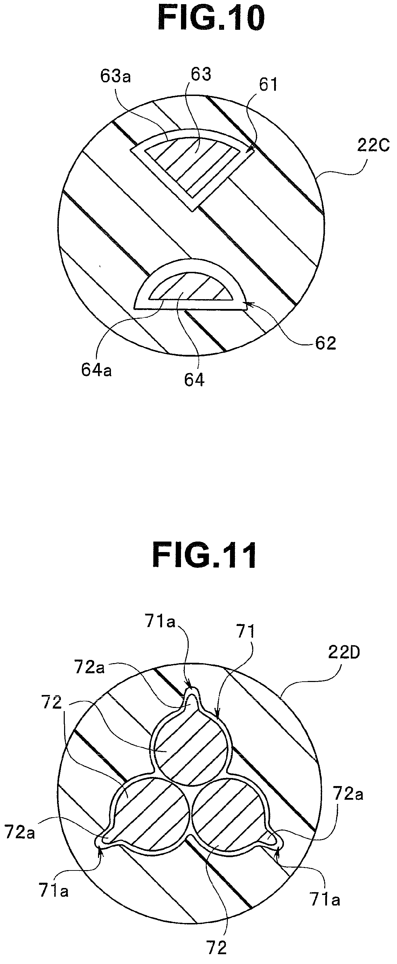

FIG. 9 and FIG. 10 illustrate other examples of the shapes of the distal-end openings of the sheath main body and the shapes of the guide wires.

FIG. 9 is a cross-sectional view of an intracorporeal insertion instrument in which a sheath main body includes four lumens. As shown in FIG. 9, a sheath main body 22B includes inside thereof four lumens 51, and is configured such that guide wires 52 are insertable into the respective lumens 51. The shapes of the distal-end openings of the sheath main body 22B are the same as the cross-sectional shapes of the lumens 51 shown in FIG. 9. That is, the shapes of the plurality of distal-end openings are different from one another.

The shape of each of the distal-end openings of the respective lumens 51 is a sector shape, and the cross-sectional shape of each of the guide wires 52 is also a sector shape analogous to but smaller than the sector shape of each of the lumens 51. A bending habit is applied to the distal end portion of each of the guide wires 52 so as to bend toward each of the cylindrical surfaces 52a.

Therefore, also the intracorporeal insertion instrument as shown in FIG. 9 enables the plurality of guide wires 52 to protrude in the directions different from each other.

FIG. 10 is a cross-sectional view of the intracorporeal insertion instrument having a plurality of lumens of different shapes. As shown in FIG. 10, a sheath main body 22C includes inside thereof two lumens 61, 62, and the lumens 61, 62 allow guide wires 63, 64 to be inserted therethrough. The shapes of the distal-end openings of the sheath main body 22C are the same as the cross-sectional shapes of the lumens 61, 62 shown in FIG. 10, respectively.

The shape of the distal-end opening of the lumen 61 is a sector shape and the cross-sectional shape of the guide wire 63 is also a sector shape analogous to but smaller than the sector shape of the lumen 61. A bending habit is applied to the distal end portion of the guide wire 63 so as to bend toward the cylindrical surface 63a.

The shape of the distal-end opening of the lumen 62 is a semicircular shape, and also the cross-sectional shape of the guide wire 64 is a semicircular shape analogous to but smaller than the semicircular shape of the lumen 62. A bending habit is applied to the distal end portion of the guide wire 64 so as to bend toward a flat surface 64a.

Therefore, also the intracorporeal insertion instrument shown in FIG. 10 enables the plurality of guide wires 63, 64 having shapes different from each other to protrude in directions different from each other.

Note that an identification tag as identification means may be provided to each of the guide wires and the sheath main body so that the plurality of guide wires having shapes different from each other can be correctly inserted into corresponding lumens, respectively.

For example, in FIG. 6, as shown by the one-dot chain lines, when the proximal end portion 22b has a sheath portion for each of the guide wires, tags 81a and 81b, each of which indicates a type, a shape, and the like of the guide wire to be inserted, are attached to the respective sheath portions, and also tags 82a and 82b, each of which indicates a type, a shape, and the like of the guide wire, are attached to the respective guide wires. For example, each of the tags is an adhesive seal or the like, on which a character, a sign or the like indicating a type, etc. is printed, and is pasted on each of the sheath portions of the proximal end portion 22b and each of the proximal ends of the guide wires by making use of the adherence property of the seal.

The tag 82a is pasted on the guide wire 63 which is one of the two guide wires having different shapes as shown in FIG. 10, for example, and when the sheath portion of the corresponding lumen 61 is a sheath portion 22A1, the tag 81a is pasted on the sheath portion 22A1. If the tags 81a and 82a have the same sign, mark and the like, the user can easily recognize that the guide wire 63 should be inserted into the sheath portion 22A1. The same is true for the guide wire 64 and the lumen 62.

Therefore, the user can easily insert the guide wires into the correct lumens by using such tags.

Furthermore, the shape of each of the distal-end openings may be a triangle shape, a trapezoid shape, a star shape, or the like, instead of the above-described semicircular shape and the sector shape.

As described above, according to the above-described embodiment and the respective modified examples, it is possible to provide an intracorporeal insertion instrument which has a simple configuration and is suitable for diameter reduction, and which enables a plurality of guide wires to protrude in directions different from each other.

### (Second Embodiment)

In the above-described embodiment, a plurality of lumens are formed in the sheath main body and the guide wires are inserted into the respective lumens. However, in the present embodiment, the sheath main body includes one lumen into which a plurality of guide wires are insertable.

FIG. 11 is a cross-sectional view of the intracorporeal insertion instrument 21 according to the present embodiment. In FIG. 11, the members corresponding to those in the above-described first embodiment are made of the same materials and produced in the same production method.

As shown in FIG. 11, a sheath main body 22D includes one lumen 71 into which three guide wires 72 are insertable.

Each of the guide wires 72 is a member having substantially circular cross section and including a protruding portion 72a along an axis direction. A bending habit is applied to each of the distal end portions of the guide wires 72 so as to bend toward the protruding portion 72a.

On the other hand, the lumen 71 is provided with three groove portions 71 a which are engaged with the protruding portions 72a of the three guide wires 72 and which are formed along the axis direction. The user such as a surgeon can insert the three guide wires 72 from the proximal-end opening of the proximal end portion 22b of the sheath main body 22D in a state where the protruding portions 72a of the respective guide wires 71 are engaged with the respective groove portions 71 a.

As shown in FIG. 11, in the lumen 71, the protruding portions 72a enter into the groove portions 71a, respectively, which prevents the three guide wires 72 from rotating in the axis direction.

That is, in the present embodiment, the sheath main body 22D includes the single lumen 71 formed such that the plurality of guide wires are inserted from the proximal-end opening provided at the proximal end portion 22b and the plurality of guide wires can be protruded from the distal-end opening provided at the distal end portion 22a. In addition, the distal-end opening is formed such that the respective guide wires are prevented from rotating around the axes of the respective guide wires in the one distal-end opening, and the plurality of the distal end portions of the plurality of guide wires are separated from each other along the protruding directions.

Therefore, the three guide wires protrude from the distal-end opening of the lumen 71 of the sheath main body 22D in directions different from each other.

Note that, also regarding the present second embodiment, the respective modified examples 1 to 3 described in the first embodiment are applicable.

The present embodiment has the same effects as those in the first embodiment, and configured such that the plurality of guide wires can be inserted into the one lumen, the diameter of the sheath main body 22D can be made smaller than the diameter of the sheath main body in the first embodiment.

As described above, according to the above-described two embodiments and the respective modified examples, it is possible to provide an intracorporeal insertion instrument which has a simple configuration and is suitable for diameter reduction, and which enables a plurality of guide wires to protrude in directions different from each other.

Note that description was made in the above-described examples by taking the intracorporeal insertion instrument for bile duct as an example. However, the above-described examples can be applied to intracorporeal insertion instruments for blood vessels of the heart, blood vessels in the brain, and the like.

The present invention is not limited to the above-described embodiments, and various changes, modifications, and the like are possible without departing from the scope of the invention.

The present application is filed claiming the priority of Japanese Patent Application No. 2011-134500 filed in Japan on June 16, 2011.

## Claims

1. An intracorporeal insertion instrument (21) configured to be inserted into a bile duct in a body when protruding from a channel of an endoscope (11), the intracorporeal insertion instrument (21) comprising:
a first elongated and flexible guide wire (25) having an insertion axis, and a distal end portion (25t) whose cross-sectional shape is a non-circular shape and to which a first bending habit is applied in advance to be bent in a predetermined direction;
a second elongated and flexible guide wire (26) having an insertion axis, and a distal end portion (26t) whose cross-sectional shape is a non-circular shape and to which a second bending habit is applied in advance to be bent in a predetermined direction;
a sheath main body (22) provided with a first lumen (23) and a second lumen (24) through which the first guide wire (25) and the second guide wire (26) are insertable, respectively, the first and second lumens (23, 24) having cross-sectional shapes complementary to cross-sectional shapes of the first and second guide wires (25, 26), respectively, such that the first and second guide wires (25, 26) are prevented from rotating around the insertion axes;
a distal end portion (22a) provided on a distal end side of the sheath main body (22) and forming a first distal-end opening (23t) of the first lumen (23) and a second distal-end opening (24t) of the second lumen (24); and
a proximal end portion (22b) provided on a proximal end side of the sheath main body (22) and forming a first proximal-end opening (23tb) of the first lumen (23) which is larger than the first distal-end opening (23t) and a second proximal-end opening (24tb) of the second lumen (24) which is larger than the second distal- end opening (24t), the proximal end portion (22b) being configured for inserting the first guide wire (25) and the second guide wire (26) such that the first guide wire (25) and the second guide wire (26) are capable of protruding from the first distal- end opening (23t) and the second distal-end opening (24t), respectively,
wherein, when the first guide wire (25) is protruded from the first distal-end opening (23t) and the second guide wire (26) is protruded from the second distal- end opening (24t), the first guide wire (25) and the second guide wire (26) are protruded to bend in directions different from each other by the first bending habit of the first guide wire (25) and the second bending habit of the second guide wire (26).

2. The intracorporeal insertion instrument according to claim 1, wherein the cross-sectional shapes of the first and second (23, 24) lumens have parts changing gradually from the first proximal-end (23tb) opening toward the first distal-end opening (23t) of the first lumen (23) and from the second proximal-end opening (24tb) toward the second distal-end opening (24t) of the second lumen (24), respectively.

3. The intracorporeal insertion instrument according to claim 2, wherein a shape of the first distal-end opening (23t) of the first lumen (23) and a shape of the second distal-end opening (24t) of the second lumen (24) are different from each other.

4. The intracorporeal insertion instrument according to claim 1, wherein the sheath main body (22) includes first and second sheath portions (21A1, 22A2) which branch off for the first lumen (23) and the second lumen (24), respectively, at the proximal end portion (22b).

5. The intracorporeal insertion instrument according to claim 4, wherein inner diameters of the first and second lumens (23, 24) are expanded gradually from the first distal-end opening (23t) toward the first proximal-end opening (23tb) and from the second distal-end opening (24t) toward the second proximal-end opening (24tb), respectively.

6. The intracorporeal insertion instrument according to claim 1, wherein the cross-sectional shape of each of the distal end portions (25t, 26t) of the first and second guide wires (25, 26) is a semicircular shape, a sector shape, a triangle shape, a trapezoidal shape, or a star shape.

7. The intracorporeal insertion instrument according to claim 1, wherein the first and second guide wires (25, 26) are solid or tubular wires.

8. The intracorporeal insertion instrument according to claim 1, wherein surfaces of the first and second guide wires (25, 26) are coated.

9. The intracorporeal insertion instrument according to claim 1, wherein the first and second guide wires (25, 26) and the sheath main body (22) are provided with a first indicator portion (41) for positioning of the first guide wire (25) in a circumferential direction of the insertion axis at the first proximal-end opening (23tb), and a second indicator portion (42) for positioning of the second guide wire (26) in a circumferential direction of the insertion axis at the second proximal-end opening (24tb).

## Patentansprüche

1. Intrakorporales Einführgerät (21), das dazu eingerichtet ist, in eine Gallenröhre in einem Körper eingeführt zu werden, wenn es von einem Kanal eines Endoskops (11) vorsteht, wobei das intrakorporale Einführgerät (21) umfasst:
einen ersten langgestreckten und flexiblen Führungsdraht (25), der eine Einführachse und einen distalen Endabschnitt (25t) hat, dessen Querschnittsform eine nicht runde Form ist und auf den eine erste Vorbiegung aufgebracht wird, bevor er in eine vorbestimmte Richtung gebogen wird;
einen zweiten langgestreckten und flexiblen Führungsdraht (26), der eine Einführachse und einen distalen Endabschnitt (26t) hat, dessen Querschnittsform eine nicht runde Form ist und auf den eine zweite Vorbiegung aufgebracht wird, bevor er in eine vorbestimmte Richtung gebogen wird;
einen Hülsenhauptkörper (22), der mit einem ersten Lumen (23) und einem zweiten Lumen (24) ausgestattet ist, durch das der erste Führungsdraht (25) beziehungsweise der zweite Führungsdraht (26) einführbar sind, wobei die ersten und zweiten Lumen (23, 24) Querschnittsformen haben, die komplementär zu den Querschnittsformen der ersten beziehungsweise zweiten Führungsdrähte (25, 26) sind, so dass, die ersten und zweiten Führungsdrähte (25, 26) daran gehindert werden, um die Einführachsen zu rotieren;
einen distalen Endabschnitt (22a), der an einer distalen Endseite des Hülsenhauptkörpers (22) vorgesehen ist und eine erste distale Endöffnung (23t) des ersten Lumens (23) und eine zweite distale Endöffnung (24t) des zweiten Lumens (24) bildet; und
einen proximalen Endabschnitt (22b), der an einer proximalen Endseite des Hülsenhauptkörpers (22) vorgesehen ist und eine erste proximale Endöffnung (23tb) des ersten Lumens (23), die größer ist als die erste distale Endöffnung (23t), und eine zweite proximale Endöffnung (24tb) des zweiten Lumens (24), die größer ist als die zweite distale Endöffnung (24t), bildet, wobei der proximale Endabschnitt (22b) dazu eingerichtet ist, den ersten Führungsdraht (25) und den zweiten Führungsdraht (26) so einzuführen, dass der erste Führungsdraht (25) und der zweite Führungsdraht (26) dazu in der Lage sind, von der ersten distalen Endöffnung (23t) beziehungsweise der zweiten distalen Endöffnung (24t) vorzustehen,
wobei, wenn der erste Führungsdraht (25) von der ersten distalen Endöffnung (23t) vorsteht und der zweite Führungsdraht (26) von der zweiten distalen Endöffnung (24t) vorsteht, der erste Führungsdraht (25) und der zweite Führungsdraht (26) vorstehen, um sich durch die erste Vorbiegung des ersten Führungsdrahts (25) und die zweite Vorbiegung des zweiten Führungsdrahts (26) in verschiedene Richtungen zu biegen.

2. Intrakorporales Einführgerät gemäß Anspruch 1, wobei die Querschnittsformen der ersten und zweiten (23, 24) Lumen Teile haben, die sich von der ersten proximalen Endöffnung (23tb) zu der ersten distalen Endöffnung (23t) des ersten Lumens (23) beziehungsweise von der zweiten proximalen Endöffnung (24tb) zu der zweiten distalen Endöffnung (24t) des zweiten Lumens (24) graduell ändern.

3. Intrakorporales Einführgerät gemäß Anspruch 2, wobei eine Form der ersten distalen Endöffnung (23t) des ersten Lumens (23) und eine Form der zweiten distalen Endöffnung (24t) des zweiten Lumens (24) verschieden voneinander sind.

4. Intrakorporales Einführgerät gemäß Anspruch 1, wobei der Hülsenhauptkörper (22) erste und zweite Hülsenabschnitte (21A1, 22A2) umfasst, die an dem proximalen Endabschnitt (22b) von dem ersten Lumen (23) beziehungsweise dem zweiten Lumen (24) abzweigen.

5. Intrakorporales Einführgerät gemäß Anspruch 4, wobei sich Innendurchmesser der ersten und zweiten Lumen (23, 24) von der ersten distalen Endöffnung (23t) zu der ersten proximalen Endöffnung (23tb) beziehungsweise von der zweiten distalen Endöffnung (24t) zu der zweiten proximalen Endöffnung (24tb) graduell erweitern.

6. Intrakorporales Einführgerät gemäß Anspruch 1, wobei die Querschnittsform eines jeden distalen Endabschnitts (25t, 26t) der ersten und zweiten Führungsdrähte (25, 26) eine Halbkreisform, eine Sektorform, eine Dreiecksform, eine trapezoidale Form oder eine Sternform ist.

7. Intrakorporales Einführgerät gemäß Anspruch 1, wobei die ersten und zweiten Führungsdrähte (25, 26) stabförmige oder röhrenförmige Drähte sind.

8. Intrakorporales Einführgerät gemäß Anspruch 1, wobei Oberflächen der ersten und zweiten Führungsdrähte (25, 26) beschichtet sind.

9. Intrakorporales Einführgerät gemäß Anspruch 1, wobei die ersten und zweiten Führungsdrähte (25, 26) und der Hülsenhauptkörper (22) mit einem ersten Indikatorabschnitt (41) zur Positionierung des ersten Führungsdrahts (25) in einer Umfangsrichtung der Einführachse der ersten proximalen Endöffnung (23tb) und einem zweiten Indikatorabschnitt (42) zur Positionierung des zweiten Führungsdrahts (26) in einer Umfangsrichtung der Einführachse der zweiten proximalen Endöffnung (24tb) ausgestattet sind.

## Revendications

1. Instrument (21) pour insertion intracorporelle configuré pour être inséré dans un canal cholédoque dans un corps lorsqu'il fait saillie hors d'un canal d'un endoscope (11), l'instrument (21) pour insertion intracorporelle comprenant :
un premier fil guide (25) allongé et flexible ayant un axe d'insertion, et une partie (25t) d'extrémité distale dont la forme en coupe transversale est une forme non circulaire et auquel un premier comportement en cintrage est appliqué à l'avance pour qu'il soit cintré dans un sens prédéterminé ;
un deuxième fil guide (26) allongé et flexible ayant un axe d'insertion, et une partie (26t) d'extrémité distale dont la forme en coupe transversale est une forme non circulaire et auquel un deuxième comportement en cintrage est appliqué à l'avance pour qu'il soit cintré dans un sens prédéterminé ;
un corps principal (22) de gaine prévu avec une première lumière (23) et une deuxième lumière (24) à travers lesquelles le premier fil guide (25) et le deuxième fil guide (26) sont insérables, respectivement, les première et deuxième lumières (23, 24) ayant des formes en coupe transversale complémentaires des formes en coupe transversale des premier et deuxième fils guides (25, 26), respectivement, de telle sorte que les premier et deuxième fils guides (25, 26) sont empêchés de tourner autour des axes de rotation ;
une partie (22a) d'extrémité distale prévue sur un côté d'extrémité distale du corps principal (22) de gaine et formant une première ouverture (23t) d'extrémité distale de la première lumière (23) et une deuxième ouverture (24t) d'extrémité distale de la deuxième lumière (24) ; et
une partie (22b) d'extrémité proximale prévue sur un côté d'extrémité proximale du corps principal (22) de gaine et formant une première ouverture (23tb) d'extrémité proximale de la première lumière (23) qui est plus grande que la première ouverture (23t) d'extrémité distale et une deuxième ouverture (24tb) d'extrémité proximale de la deuxième lumière (24) qui est plus grande que la deuxième ouverture (24t) d'extrémité distale, la partie (22b) d'extrémité proximale étant configurée pour insérer le premier fil guide (25) et le deuxième fil guide (26) de telle sorte que le premier fil guide (25) et le deuxième fil guide (26) sont apte à faire saillie de la première ouverture (23t) d'extrémité distale et de la deuxième ouverture (24t) d'extrémité distale, respectivement,
dans lequel, lorsque le premier fil guide (25) fait saillie de la première ouverture (23t) d'extrémité distale et que le deuxième fil guide (26) fait saillie de la deuxième ouverture (24t) d'extrémité distale, le premier fil guide (25) et le deuxième fil guide (26) font saillie de façon à être cintrés dans des directions différentes l'un par rapport à l'autre par le premier comportement en cintrage du premier fil guide (25) et le deuxième comportement en cintrage du deuxième fil guide (26).

2. Instrument pour insertion intracorporelle selon la revendication 1, dans lequel les formes en coupe transversale des première et deuxième lumières (23, 24) ont des parties changeant graduellement de la première ouverture (23tb) d'extrémité proximale vers la première ouverture (23t) d'extrémité distale de la première lumière (23) et de la deuxième ouverture (24tb) d'extrémité proximale vers la deuxième ouverture (24t) d'extrémité distale de la deuxième lumière (24), respectivement.

3. Instrument pour insertion intracorporelle selon la revendication 2, dans lequel une forme de la première ouverture (23t) d'extrémité distale de la première lumière (23) et une forme de la deuxième ouverture (24t) d'extrémité distale de la deuxième lumière (24) sont différentes l'une de l'autre.

4. Instrument pour insertion intracorporelle selon la revendication 1, dans lequel le corps principal (22) de gaine inclut des première et deuxième parties (21A1, 22A2) de gaine qui partent de la première lumière (23) et de la deuxième lumière (24), respectivement, au niveau de la partie (22b) d'extrémité proximale.

5. Instrument pour insertion intracorporelle selon la revendication 4, dans lequel des diamètres intérieurs des première et deuxième lumières (23, 24) sont agrandis graduellement de la première ouverture (23t) d'extrémité distale vers la première ouverture (23tb) d'extrémité proximale et de la deuxième ouverture (24t) d'extrémité distale vers la deuxième ouverture (24tb) d'extrémité proximale, respectivement.

6. Instrument pour insertion intracorporelle selon la revendication 1, dans lequel la forme en coupe transversale de chacune des parties (25t, 26t) d'extrémité distale des premier et deuxième fils guides (25, 26) est une forme semi-circulaire, une forme sectorielle, une forme triangulaire, une forme trapézoïdale ou une forme en étoile.

7. Instrument pour insertion intracorporelle selon la revendication 1, dans lequel les premier et deuxième fils guides (25, 26) sont des fils pleins ou tubulaires.

8. Instrument pour insertion intracorporelle selon la revendication 1, dans lequel des surfaces des premier et deuxième fils guides (25, 26) sont revêtues.

9. Instrument pour insertion intracorporelle selon la revendication 1, dans lequel les premier et deuxième fils guides (25, 26) et le corps principal (22) de gaine sont prévus avec une première partie (41) d'indicateur pour positionner le premier fil guide (25) dans un sens circonférentiel de l'axe d'insertion au niveau de la première ouverture (23tb) d'extrémité proximale, et une deuxième partie (42) d'indicateur pour positionner le deuxième fil guide (26) dans un sens circonférentiel de l'axe d'insertion au niveau de la deuxième ouverture (24tb) d'extrémité proximale.
